Europäisches Patentamt

European Patent Office

Office européen des brevets

(19)

(11) Numéro de publication: **0 385 884**
**A1**

(12) **DEMANDE DE BREVET EUROPEEN**

(21) Numéro de dépôt: **90420070.6**

(22) Date de dépôt: **09.02.90**

(51) Int. Cl.5: **C07C 201/08, C07C 205/06**

(30) Priorité: **21.02.89 FR 8902468**

(43) Date de publication de la demande:
**05.09.90 Bulletin 90/36**

(84) Etats contractants désignés:
**BE DE ES FR GB IT NL**

(71) Demandeur: **RHONE-POULENC CHIMIE**
**25, quai Paul Doumer**
**F-92408 Courbevoie Cédex(FR)**

(72) Inventeur: **Gubelmann, Michel**
**39, Bd des Belges**
**F-69006 Lyon(FR)**
Inventeur: **Popa, Jean-Michel**
**2, rue Roger Breton**
**F-93700 Drancy(FR)**
Inventeur: **Tirel, Philippe-Jean**
**40, Bd Kennedy**
**F-69600 Oullins(FR)**
Inventeur: **Quemere, Eric**
**11bis, rue Martray**
**F-95240 Cormeilles en Parisis(FR)**
Inventeur: **Doussain, Claude**
**2, rue Louis Girardet**
**F-69190 Saint-Fons(FR)**

(74) Mandataire: **Varnière-Grange, Monique et al**
**RHONE-POULENC CHIMIE Service Brevets**
**Chimie Centre de Recherches des Carrières**
**B.P. 62**
**F-69192 Saint-Fons Cédex(FR)**

(54) **Procédé de préparation de dinitrotoluènes.**

(57) La présente invention concerne un procédé de préparation du dinitrotoluène par réaction du mononitrotoluène et de l'acide nitrique en phase liquide. Le procédé est caractérisé en ce que la réaction est conduite en présence d'un sulfate métallique et le cas échéant en présence d'un diluant choisi parmi les (poly) cycloalcanes perfluorés comportant de 5 à 12 atomes de carbone et le perfluorotoluène.

## PROCEDE DE PREPARATION DE DINITROTOLUENES

La présente invention concerne un procédé de préparation de dinitrotoluènes. Elle a plus particulièrement trait à un procédé de préparation de dinitrotoluènes par réaction d'au moins un mononitroluène et de l'acide nitrique en phase liquide en présence d'un sulfate métallique.

Les dinitrotoluènes, en particulier le dinitro-2,4 toluène, le cas échéant en mélange avec le dinitro-2,6 toluène, sont des produits intermédiaires de la chaîne de fabrication du toluène-diisocyanate (TDI) qui entre dans la préparation de divers polyuréthanes. Le dinitro-2,4 toluène est donc produit industriellement et à une très grande échelle. Dans un tel contexte il est aisé de concevoir l'importance que peut revêtir une augmentation de quelques pourcents sur un taux de conversion et/ou sur une sélectivité, par exemple, ou une amélioration au niveau de l'une quelconque des étapes de mise en oeuvre du procédé.

Le dinitro-2,4 toluène est classiquement produit par une double nitration en phase liquide du toluène au moyen d'un mélange d'acide nitrique et d'acide sulfurique concentré. La concentration de l'acide sulfurique utilisé pour la deuxième nitration (nitration du mononitrotoluène en dinitrotoluène) étant typiquement de l'ordre de 96 %. Nonobstant les problèmes de corrosion et de coûts importants du recyclage de grands volumes d'acide sulfurique, le procédé présente des inconvénients importants que sont le caractère problèmatique de l'élimination du catalyseur, résolu généralement par des lavages à l'eau, et la nécessité de concentrer l'acide sulfurique avant son recyclage dans la mesure où la réaction elle-même génère de l'eau.

Diverses tentatives, en particulier pour ce qui concerne la nitration sélective du toluène en position para, ont été évoquées dans le préambule du brevet américain n° 3,957,889 dans lequel il est proposé de conduire la nitration de certains composés aromatiques le cas échéant, substitués par un atome d'halogène, un groupe halogénoalkyle, nitro ou méthoxy, en présence de sulfate de calcium anhydre, produit parfois désigné par le terme d'anhydrite soluble.

Il est également préconisé dans ce document antérieur d'utiliser un diluant tel le chloroforme, le chlorure de méthylène, le nitrotoluène et le recours à un diluant dans lequel les réactifs sont hautement solubles est présenté comme étant préférable pour une bonne mise en oeuvre du procédé en cause.

Toutefois s'agissant de la nitration d'un substrat à faible réactivité tel l'orthonitrotoluène le procédé préconisé ne paraît pas pleinement satisfaisant dans la mesure où d'une part la sélectivité en isomère 2,4 est insuffisante et, d'autre part, l'activité (ou le rendement) reste négligeable.

C'est pourquoi il serait souhaitable de pouvoir disposer d'un procédé efficace de préparation du dinitrotoluène qui permette d'obvier au moins partiellement aux inconvénients précités.

Il a maintenant été trouvé que d'autres sulfates métalliques présentent une activité supérieure à celle exercée par le sulfate de calcium anhydre.

La présente invention a pour objet, dans un premier aspect, un procédé de préparation de dinitrotoluène(s) par réaction d'au moins un mononitrotoluène et de l'acide nitrique en phase liquide caractérisé en ce que la réaction est conduite en présence d'un sulfate métallique choisi dans le groupe constitué par les sulfates d'un ou plusieurs métaux mono-, di- ou trivalents, à l'exclusion du sulfate simple de calcium.

Il a également été trouvé que certains diluants dans lesquels les réactifs sont peu ou pas solubles permettent d'améliorer l'activité de nitration et le cas échéant la sélectivité en l'isomère plus particulièrement recherché.

La présente invention a donc également pour objet, dans son second aspect un procédé de préparation de dinitrotoluènes par réaction d'au moins un mononitrotoluène et de l'acide nitrique en phase liquide caractérisé en ce que la réaction est conduite en présence d'un sulfate métallique et d'un diluant choisi dans le groupe constitué par les (poly) cycloalcanes perfluorés comportant de 5 à 12 atomes de carbone et le perfluorotoluène.

Par dinitrotoluène(s) on entend, de préférence, dans le cadre du présent procédé le dinitro-2,4 toluène, le dinitro-2,6 toluène et leurs mélanges.

Par mononitrotoluène(s) on entend le p-nitrotoluène, l'o-nitrotoluène et leurs mélanges.

Bien entendu, si la matière de départ est un mélange d'ortho- et de para-nitrotoluène on obtiendra un mélange d'isomères 2,4 et 2,6 du dinitrotoluène ; si la matière de départ est le para-nitrotoluène, le dinitro-2,4 toluène sera le seul produit observé.

Le procédé selon l'invention requiert la présence d'acide nitrique. On recourt généralement à des formes concentrées d'acide nitrique, la concentration étant supérieure à 90 % et de préférence, comprise entre 95 et 100 %.

L'une des caractéristiques essentielles du présent procédé réside dans l'utilisation d'un sulfate métalli-

2

que.

Par sulfates métalliques on entend les sels d'un ou plusieurs métaux susceptibles de former des cations porteurs de 1 à 3 charges positives et de l'acide sulfurique dans lequel au moins un atome d'hydrogène a été remplacé par un atome métallique, sels simples ou mixtes, qui se présentent sous forme d'hydrates plus ou moins pauvres en eau selon que la température de conditionnement est plus ou moins élevée ; dans le cadre du présent procédé, on engagera des formes partiellement ou totalement déshydratées desdits sulfates. Les sels métalliques susceptibles d'être utilisés dans le cadre du présent procédé sont en général des hydrates contenant au plus la quantité d'eau correspondant à l'hydrate stable à température ambiante. Des exemples de tels hydrates sont indiqués dans la colonne II du tableau ci-après.

On recourt de préférence aux hydrates de tels sels contenant au plus la quantité d'eau correspondant à l'hydrate défini le moins riche en eau ou au sel anhydre, quand ce dernier peut être obtenu, est thermiquement stable et garde des propriétés de réhydratation. Des exemples de tels sulfates sont indiqués dans la colonne III du tableau ci-après.

A titre d'exemples de tels sulfates on peut citer :

| I | II | III |
|---|---|---|
| SULFATE | HYDRATES UTILISABLES | SELS ANHYDRES OU HYDRATES PREFER. UTILISES |
| $MgSO_4, x\ H_2O$ | $x \leq 7$ | $O \leq x \leq 0,5$ |
| $CaSO_4, y\ H_2O$ | $y \leq 2$ | $O \leq y \leq 0,5$ |
| $CuSO_4, z\ H_2O$ | $z \leq 5$ | $O \leq z \leq 1$ |
| $NiSO_4, t\ H_2O$ | $t \leq 6$ | $O \leq t \leq 1$ |
| $La_2(SO_4)_3, n\ H_2O$ | $n \leq 9$ | $O \leq n \leq 2$ |
| $Al_2(SO_4)_3, p\ H_2O$ | $p \leq 18$ | $O \leq p \leq 3$ |

On recourt avantageusement à des sulfates non acides ou exempts d'atomes d'hydrogène et de préférence à des sulfates des métaux susceptibles de former des cations porteurs de 2 ou 3 charges positives.

Les sulfates de lanthane, de cuivre, de magnésium et de calcium conviennent plus particulièrement bien à la mise en oeuvre du présent procédé.

Les sulfates métalliques peuvent se présenter sous des formes massiques ou dispersées telles que billes, pastilles et poudres dont les dimensions moyennes sont généralement comprises entre 0,1 micromètre et 10 mm.

La quantité optimale de sulfate métallique à mettre en oeuvre sera choisie de telle manière que le rapport molaire (eau théoriquement libérable par la nitration)/sulfate métallique soit compris dans l'intervalle donné pour l'hydrate défini le moins riche en eau, des exemples de tels intervalles figurant colonne (III) du tableau ci-avant.

Bien entendu, on ne sort pas du cadre de la présente invention en s'écartant de ladite quantité optimale par excès ou par défaut. La Demanderesse préconise plus particulièrement de ne pas s'écarter de cette valeur optimale (par excès ou par défaut) de plus de 20 % et, de préférence, de plus de 10 %.

Dans son premier aspect exposé, l'invention n'entend pas recourir au seul sulfate simple de calcium ; ceci n'exclut cependant pas le recours à un sulfate mixte renfermant du calcium et un ou plusieurs autres métaux.

Dans son second aspect exposé, la présente invention comporte deux caractéristiques essentielles à savoir la présence d'un sulfate métallique et celle d'un diluant particulier. Par sulfate métallique on entend dans ce cadre les sels définis précédemment, incluant le sulfate simple de calcium. La seconde caractéristique essentielle réside dans le fait que la réaction de nitration est conduite dans un diluant choisi dans le groupe constitué par les (poly) cycloalcanes perfluorés comportant de 5 à 12 atomes de carbone et le perfluorotoluène.

Parmi les (poly) cycloalcanes perfluorés on citera plus particulièrement les composés dérivant respectivement du cyclohexane ou du méthyl-cyclohexane par remplacement de tous les atomes d'hydrogène au moyen d'atomes de fluor (à savoir le perfluorocyclohexane et le perfluoro méthylcyclohexane) et la perfluorodécaline.

On recourt avantageusement à la perfluorodécaline Pour une bonne mise en oeuvre, la Demanderesse préconise l'utilisation de 0,5 à 5 ml de diluant par g de sulfate engagé, des rapports différents pouvant être utilisés en pratique pour des raisons économiques et/ou des raisons liées à la conception même du réacteur choisi pour assurer un bon contact liquide-solide.

La réaction est généralement conduite dans un dispositif assurant un bon contact liquide-solide tel un réacteur agité ou un réacteur avec boucle de circulation de liquide.

La réaction entre le(s) mononitrotoluène(s) et l'acide nitrique est conduite en phase liquide, le rapport molaire de l'acide nitrique au(x) mononitrotoluène(s) pouvant varier dans de larges limites. Pour une bonne mise en oeuvre le rapport molaire sera compris entre 0,1 et 10.

En général, une température d'au moins 20°C s'avère nécessaire pour obtenir une vitesse de conversion acceptable et au-delà de 100°C, des réactions parasites sont susceptibles de se produire et de diminuer l'intérêt d'un tel procédé.

Au bout du temps fixé pour la réaction (ou du temps de séjour souhaité) on récupère le(s) dinitrotoluène(s) par tout moyen approprié, par exemple par distillation le cas échéant précédée par la décantation ou la filtration du sulfate métallique.

Les exemples ci-après illustrent l'invention.

EXEMPLE 1 . Essais témoins (a) et (b)

Dans un réacteur en verre de 30 ml de capacité, équipé d'une agitation magnétique, on charge :
. 9,1 mmol d'orthonitrotoluène le cas échéant, 2,5 ml de diluant,
. 2,5 g de Ca SO₄ anhydre obtenu par chauffage à 250°C pendant 3 h de CaSO₄,2H₂O
. 9,4 mmol d'acide nitrique (100 %)

Le mélange est maintenu à 25°C pendant 17 heures sous agitation. Puis, le mélange résultant est filtré ; le filtrat est lavé avec de l'eau.

La phase organique est analysée par chromotographie en phase gazeuse. Les conditions particulières ainsi que les résultats obtenus figurent dans le tableau I ci-après dans lequel :
PFD désigne la perfluorodécaline
TT désigne le taux de transformation de l'o-nitrotoluène
RT désigne le rendement en dinitrotoluènes par rapport à l'o-nitrotoluène.
Rép. Isom. représente la répartition isomérique
+ indique la présence de sulfate de calcium.

TABLEAU I

| Ref | diluant | | TT (%) | RT (%) | Rép. Isom. % | |
|-----|---------|-----|--------|--------|---------|---------|
| | | | | | 2,4 DNT | 2,6 DNT |
| a | CHCl₃ | | ~ 5 | 80 | 38 | 62 |
| b | CHCl₃ | + | 17,5 | 80 | 70 | 30 |
| 1 | PFD | + | 58 | 95 | 68 | 32 |

EXEMPLES 2 à 9 . Essai Témoin(c):

Dans un ballon tricol de 50 cm³ muni d'une agitation centrale et d'un réfrigérant on introduit, sauf mention contraire, 4 g de sulfate dont la nature, la provenance commerciale et, le cas échéant, les conditions de traitement préalable sont précisées dans le tableau II ci-après, le volume indiqué de perfluorodécaline (PFD ; C₁₀ F₁₈) sauf mention contraire, 1,37 g (10 mmol) de para-nitrotoluène, 0,43 cm³ (10,4 mmol) d'acide nitrique à 100 %. Le mélange est porté à 60°C pendant 1 heure (sauf mention contraire), sous agitation.

Le mélange résultant est filtré ; le filtrat est lavé avec de l'eau.

La phase organique est alors analysée par chromatographie en phase gazeuse.

Les conditions particulières ainsi que les résultats obtenus figurent dans le tableau II ci-après dans lequel

TT désigne le taux de transformation du p-nitrotoluène

RT désigne le rendement en dinitro-2,4 toluène (DNT).

TABLEAU II

| Réf. | PFD cm$^3$ | SULFATE | | engagé | TT (%) | RT (%) |
|------|-----------|---------|---|--------|--------|--------|
| | | nature et origine | traitement | | | |
| c (*) | 8 | Néant | - | - | 27 | 76 |
| 2 | 5 | Mg SO$_4$ . 7H$_2$O (Prolabo) | 200°C - 3 h | MgSO$_4$ . 0,2H$_2$O | 57 | 94 |
| | | | 650°C - 2 h | | | |
| 3 (**) | 5 | Ca SO$_4$ . 2H$_2$O (Prolabo) | 250°C - 3 h | CaSO$_4$ | 51 | 91 |
| 4 | 5 | Ca SO$_4$ . 2H$_2$O (Prolabo) | 250°C - 3 h | CaSO$_4$ | 48 | ~ 100 |
| 5 (*) | 5 | Ca SO$_4$ . 2H$_2$O (Prolabo) | 250°C - 3 h | CaSO$_4$ | 61 | ~ 100 |
| 6 | 3,5 | Cu SO$_4$ . 5H$_2$O (Prolabo) | 300°C - 4 h | CuSO$_4$ | 71 | 98 |
| 7 | 5 | La$_2$ (SO$_4$)$_3$.8H$_2$O (Ventron) | 300°C - 4 h | La$_2$(SO$_4$)$_3$ | 54 | 95 |
| 8 (a) | 5 | CaSO$_4$.2H$_2$O (Prolabo) | 250°C - 3 h | CaSO$_4$ | 56 | 95 |
| 9 (b) | 5 | CaSO$_4$.2H$_2$O (Prolabo) | 250°C - 3 h | CaSO$_4$ | 51 | 96 |
| NB : | | | | | | |

(*) durée : 3,5 heures

(**) 2 g de sulfate engagé

- (a) le diluant est le méthylcyclohexane perfluoré

- (b) le diluant est le composé perfluoré FC 72 (commercial)

EXEMPLE 10:

Dans l'appareillage et selon le mode opératoire, décrits pour l'exemple 1 ci-avant on réalise un essai sur une charge constituée par :

922 mg (6 mmol) d'o-nitrotoluène

549 mg (4 mmol) de p-nitrotoluène

4 cm$^3$ de PFD

4 g de Ca SO$_4$ anhydre

0,43 cm$^3$ (10,6 mmol) d'HNO$_3$ à 100 %.

En 1 h 30 mn de réaction à 60°C on obtient les résultats suivants :

Taux de transformation global des nitrotoluènes : 68,8 % Rendement global en dinitrotoluènes (2,4 et 2,6) par rapport aux nitrotoluènes : 92,9 %

Taux de transformation de l'o-nitrotoluène : 77 %

Taux de transformation du p-nitrotoluène : 56 %

Répartition Isomérique

- 2,4 - dinitrotoluène : 79 %

- 2,6 - dinitrotoluène : 21 %

**Revendications**

1. Procédé de préparation de dinitrotoluène(s) par réaction d'au moins un mononitrotoluène et de l'acide nitrique en phase liquide caractérisé en ce que la réaction est conduite en présence d'un sulfate métallique choisi dans le groupe constitué par les sulfates d'un ou plusieurs métaux mono-,di- ou trivalents, à l'exclusion du sulfate simple de calcium.

2. Procédé de préparation de dinitrotoluène(s) par réaction d'au moins un mononitrotoluène et de l'acide nitrique en phase liquide caractérisé en ce que la réaction est conduite en présence d'un sulfate métallique et d'un diluant choisi dans le groupe constitué par les (poly)cycloalcanes perfluorés comportant de 5 à 12 atomes de carbone et le perfluorotoluène.

3. Procédé selon la revendication 1 ou 2, caractérisé en ce que le sulfate métallique est choisi parmi les formes partiellement ou totalement déshydratées des sels des métaux susceptibles de former des cations porteurs de 1 à 3 charges positives et de l'acide sulfurique.

4. Procédé selon la revendication 3 caractérisé en ce que le sulfate métallique est choisi parmi les formes partiellement ou totalement déshydratées des sels des métaux susceptibles de former des cations porteurs de 2 ou 3 charges positives et de l'acide sulfurique.

5. Procédé selon l'une quelconque des revendications 2 à 4, caractérisé en ce que le diluant est la perfluorodécaline.

6. Procédé selon l'une quelconque des revendications précédentes caractérisé en ce que la quantité de sulfate est telle que le rapport molaire (eau théoriquement libérable par la nitration)/sulfate métallique soit compris dans l'intervalle donné pour l'hydrate défini le moins riche en eau à ± 20 % près.

7. Procédé selon la revendication 6, caractérisé en ce que la quantité de sulfate est telle que le rapport molaire (eau théoriquement libérable par la nitration)/sulfate métallique soit compris dans l'intervalle donné pour l'hydrate défini le moins riche en eau à ± 10 % près.

8. Procédé selon l'une quelconque des revendications précédentes, caractérisé en ce que le rapport molaire $HNO_3$/mononitrotoluène(s) est compris entre 0,1 et 10.

9. Procédé selon l'une quelconque des revendications précédentes, caractérisé en ce que la température de réaction est comprise entre 20 et 100°C.

10. Procédé selon l'une quelconque des revendications 2 à 9 caractérisé en ce que le sulfate est choisi parmi les sulfates de calcium, de cuivre, de lanthane et de magnésium.

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.⁵) |
|---|---|---|---|
| A,D | US - A - 3957889 (B. MILLIGAN et al.)<br>* résumé; colonne 7, exemple 4; colonne 7, tableau 2 *<br>--- | 1 | C07C201/08<br>C07C205/06 |
| A | FR - A - 2358381 (HOECHST AG)<br>* page 3, lignes 4-12; revendications *<br>--- | 1,3,4 | |
| A | DE - A - 2510095 (TEIJIN LTD)<br>* pages 17-20; revendications 1,3 *<br>--- | 1,3,4 | |
| A | CHEMICAL ABSTRACTS<br>vol. 100, no. 11, 12 mars 1984, page 516, abrégé no. 85383h, Columbus, Ohio, USA;<br>& JP - A - 58 185 543 (SUMITOMO) 29.10.83<br>--- | 1,3 | |
| A | PATENT ABSTRACTS OF JAPAN<br>vol. 8, no. 46 (C-212)(1483), 29 février 1984; & JP - A - 58 203 946 (MITSUI TOATSU) 28.11.1983<br>--- | 2 | |
| A | FR - A - 2202056 (BASF AG)<br>* pages 3,4; revendication 1 *<br>--- | 2 | DOMAINES TECHNIQUES RECHERCHES (Int. Cl.⁵) |
| A | US - A - 3708546 (CLIFFORD L. COON et al.)<br>* le document en entier *<br>--- | 2 | C07C201/00<br>C07C205/00 |

Le présent rapport de recherche a été établi pour toutes les revendications

| Lieu de la recherche | Date d'achèvement de la recherche | Examinateur |
|---|---|---|
| Berlin | 02.06.1990 | J. RUFET |

Office européen

des brevets

## REVENDICATIONS DONNANT LIEU AU PAIEMENT DE TAXES

La présente demande de brevet européen comportait lors de son dépôt plus de dix revendications.

☐ Toutes les taxes de revendication ayant été acquittées dans les délais prescrits, le présent rapport de recherche européenne a été établi pour toutes les revendications.

☐ Une partie seulement des taxes de revendication ayant été acquittée dans les delais prescrits, le present rapport de recherche europeenne a été établi pour les dix premieres revendications ainsi que pour celles pour lesquelles les taxes de revendication ont été acquittees.

à savoir les revendications:

☐ Aucune taxe de revendication n'ayant été acquittee dans les délais prescrits, le présent rapport de recherche européenne a été établi pour les dix premières revendications.

## ABSENCE D'UNITE D'INVENTION

La division de la recherche estime que la presente demande de brevet europeen ne satisfait pas à l'exigence relative a l'unite d'invention et concerne plusieurs inventions ou pluralités d'inventions.

à savoir:

```
1. revendication: 1,3,4,
                  6-9     Nitratien en phase liquide en
                          présence de sulfate métallique,
                          à l'exception du sulfate de
                          calcium
2. revendication: 2,5,10 Nitratien en présence de sulfate
                          metallique et d'un diluant
```

☐ Toutes les nouvelles taxes de recherche ayant ete acquittees dans les delais impartis, le present rapport de recherche europeenne a ete etabli pour toutes les revendications.

☐ Une partie seulement des nouvelles taxes de recherche ayant ete acquittee dans les delais impartis, le present rapport de recherche europeenne a ete etabli pour les parties de la demande de brevet europeen qui se rapportent aux inventions pour lesquelles les taxes de recherche ont ete acquittees

à savoir les revendications:

☐ Aucune nouvelle taxe de recherche n'ayant ete acquittee dans les delais impartis, le present rapport de recherche europeenne a ete etabli pour les parties de la demande de brevet europeen qui se rapportent à l'invention mentionnee en premier lieu dans les revendications.

à savoir les revendications: